(19)

**Europäisches Patentamt**
**European Patent Office**
**Office européen des brevets**

(11) **EP 4 338 731 A1**

(12) **EUROPEAN PATENT APPLICATION**
published in accordance with Art. 153(4) EPC

(43) Date of publication:
**20.03.2024 Bulletin 2024/12**

(21) Application number: **22807456.3**

(22) Date of filing: **10.05.2022**

(51) International Patent Classification (IPC):
*A61K 31/194* (2006.01)     *A23L 33/10* (2016.01)
*A61K 9/20* (2006.01)       *A61P 13/12* (2006.01)
*A61P 17/00* (2006.01)      *A61P 19/02* (2006.01)
*A61P 21/00* (2006.01)

(52) Cooperative Patent Classification (CPC):
**A23L 33/10; A61K 9/20; A61K 31/194;**
**A61P 13/12; A61P 17/00; A61P 19/02; A61P 21/00**

(86) International application number:
**PCT/JP2022/019747**

(87) International publication number:
**WO 2022/239755 (17.11.2022 Gazette 2022/46)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **10.05.2021  JP 2021080076**

(71) Applicants:
• **Tohoku University**
  **Sendai-shi, Miyagi 980-8577 (JP)**
• **Nippon Chemiphar Co., Ltd.**
  **Tokyo 101-0032 (JP)**

(72) Inventors:
• **ABE Michiaki**
  **Sendai-shi, Miyagi 980-8577 (JP)**

• **AKAISHI Tetsuya**
  **Sendai-shi, Miyagi 980-8577 (JP)**
• **ISHIZAWA Kota**
  **Sendai-shi, Miyagi 980-8577 (JP)**
• **NAKAI Toshiki**
  **Tokyo 101-0032 (JP)**
• **KAWAGUCHI Kazuhiko**
  **Tokyo 101-0032 (JP)**
• **YAMASAKI Satomi**
  **Tokyo 101-0032 (JP)**
• **NISHIOKA Koichiro**
  **Tokyo 101-0032 (JP)**
• **SAKURAI Tetsuya**
  **Tokyo 101-0032 (JP)**

(74) Representative: **Kraus & Lederer PartGmbB et al**
**Thomas-Wimmer-Ring 15**
**80539 München (DE)**

(54) **COMPOSITION FOR INCREASING MUSCLE MASS**

(57)     Provided is a composition for increasing muscle mass in a mammal, the composition including citric acid, a salt of citric acid, a hydrate thereof, or a mixture thereof.

EP 4 338 731 A1

**(Cont. next page)**

# FIG. 1

**muscle relative weight**

Mean±SD

*p=0.0306 (unpaired t test)

$$\text{relative weight [g/100 g B.W.]} = \frac{\text{muscle weight [g]}}{\text{body weight [g]}} \times 100$$

**Description**

[Technical Field]

The present invention relates to the use of citric acid, a salt thereof, a hydrate thereof, or a mixture thereof, for increasing muscle mass, and the like.
Priority is claimed on Japanese Patent Application No. 2021-80076, filed May 10, 2021, the content of which is incorporated herein by reference.

[Background Art]

When bed rest is continued for a certain period of time due to disease or injury, muscle strength decreases, and muscles atrophy. Disuse syndrome is known as a disorder caused by such an inactive state of the body. When affected by disuse syndrome, it is difficult for patients to recover the original state, and there is a high possibility of being in a so-called bedridden state. Particularly in aged people, it is said that the disuse syndrome progresses rapidly, and 20% of the muscles of the lower limbs atrophy in two weeks of bed rest. In recent years, along with the rapid progress of an aging society, prevention of disuse syndrome has become more important. Under such circumstances, there is a demand for a safe food composition capable of suppressing muscle atrophy and increasing the muscle mass.
Patent Document 1 discloses a food composition for suppressing disuse muscle atrophy, the food composition containing naringenin as an active ingredient.
Patent Document 2 discloses a food composition for increasing muscle mass, the food composition containing lemon juice, oligosaccharide acid, and calcium as active ingredients.

[Citation List]

[Patent Documents]

[Patent Document 1] JP 2016-136952 A
[Patent Document 2] JP 2018-153166 A

[Summary of Invention]

[Technical Problem]

An object of the present invention is to provide a composition useful for increasing muscle mass. Another object of the present invention is to provide a composition useful for preventing disuse syndrome. Another object of the present invention is to provide a composition useful for the rehabilitation of a long-term bedridden person.

[Solution to Problem]

The inventors of the present invention conducted a thorough investigation to achieve the above-described objects, and they found that citric acid, a salt of citric acid, a hydrate thereof, or a mixture thereof increases the muscle mass, thus completing the invention.
In one aspect, the present invention provides a composition for increasing muscle mass, the composition including citric acid, a salt of citric acid, a hydrate thereof, or a mixture thereof.
In one aspect, the present invention provides a composition for preventing disuse syndrome, the composition including citric acid, a salt of citric acid, a hydrate thereof, or a mixture thereof.
In one aspect, the present invention provides a composition for enhancing the effect of rehabilitation of a long-term bedridden person, the composition including citric acid, a salt of citric acid, a hydrate thereof, or a mixture thereof.
In one aspect, the present invention provides a composition for suppressing a decrease in the muscle mass of an aged person, the composition including citric acid, a salt of citric acid, a hydrate thereof, or a mixture thereof.
That is, the present invention has the following aspects.

(1) A composition for increasing muscle mass in a mammal, the composition including citric acid, a salt of citric acid, a hydrate thereof, or a mixture thereof.
(2) The composition according to (1), in which the composition is a food composition.

(3) The composition according to (1) or (2), in which the citric acid, the salt of citric acid, the hydrate thereof, or the mixture thereof is an alkali metal salt of citric acid.

(4) The composition according to any one of (1) to (3), in which the citric acid, the salt of citric acid, the hydrate thereof, or the mixture thereof is a mixture of sodium citrate or a hydrate thereof and potassium citrate or a hydrate thereof.

(5) The composition according to (3) or (4), further including anhydrous citric acid.

(6) The composition according to any one of (1) to (5), in which the composition is a food composition in which an effect of increasing muscle mass is indicated on packaging, a container, or an instruction leaflet.

(7) The composition according to any one of (1) to (6), in which the composition is used for preventing disuse syndrome.

(8) The composition according to any one of (1) to (7), in which the composition is used for enhancing an effect of rehabilitation of a long-term bedridden person.

(9) The composition according to any one of (1) to (8), in which the composition is used for suppressing a decrease in muscle mass of an aged person.

(10) The composition according to any one of (1) to (9), in which the composition is a tablet.

(11) The composition according to any one of (1) to (10), in which the composition ameliorates a renal function of a mammal.

(12) The composition according to any one of (1), (3) to (5), and (7) to (11), in which the composition is a pharmaceutical composition.

[Effects of Invention]

The muscle mass is increased by taking in or ingesting a pharmaceutical composition or a food composition provided by the present invention.

[Brief Description of Drawings]

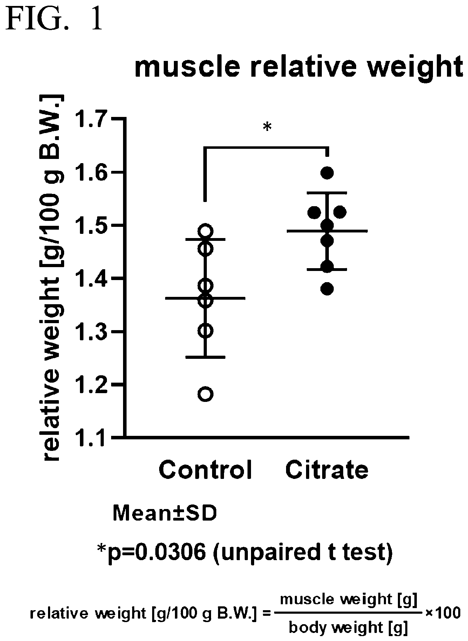

FIG. 1 is a graph showing the relative muscle masses of vastus lateralis muscle (weight of vastus lateralis muscle per 100 g of body weight) of rats to which a mixture of sodium citrate, potassium citrate, and citric acid was administered, and rats to which purified water was administered. Each bar indicates the average value and the standard deviation.

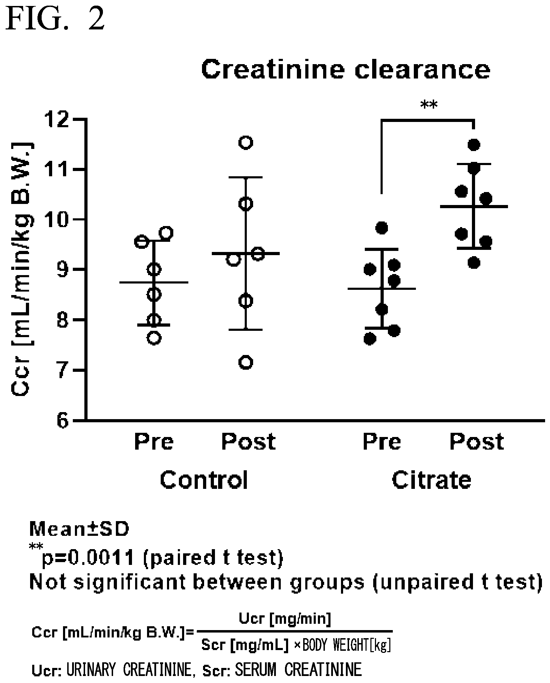

FIG. 2 is a graph showing creatinine clearances before administration and after administration in rats to which a mixture of sodium citrate, potassium citrate, and citric acid was administered, and rats to which purified water was administered. Each bar indicates the average value and the standard deviation.

[Description of Embodiments]

A composition provided by the present invention can include citric acid, a salt of citric acid, a hydrate thereof, or a mixture thereof, as an active ingredient. The salt of citric acid may be a salt acceptable as a medicine or a food. Examples of the salt of citric acid include an alkali metal salt of citric acid. Examples of the alkali metal salt of citric acid include potassium citrate and sodium citrate, and a mixture thereof may also be used. Examples of potassium citrate and sodium citrate may include hydrates such as tripotassium citrate monohydrate ($C_6H_5K_3O_7 \cdot H_2O$) and trisodium citrate dihydrate ($C_6H_5Na_3O_7 \cdot 2H_2O$), each of which is stable.

Preferred examples of the active ingredient include anhydrous citric acid, sodium citrate, potassium citrate, a hydrate thereof, and a mixture thereof, and the active ingredient may be, for example, a mixture of tripotassium citrate monohydrate ($C_6H_5K_3O_7 \cdot H_2O$) and trisodium citrate dihydrate ($C_6H_5Na_3O_7 \cdot 2H_2O$); or a mixture of tripotassium citrate monohydrate ($C_6H_5K_3O_7 \cdot H_2O$), trisodium citrate dihydrate ($C_6H_5Na_3O_7 \cdot 2H_2O$), and anhydrous citric acid.

A mixing ratio of tripotassium citrate monohydrate ($C_6H_5K_3O_7 \cdot H_2O$) and trisodium citrate dihydrate ($C_6H_5Na_3O_7 \cdot 2H_2O$) can be appropriately set by a person skilled in the art, and for example, a molar ratio of tripotassium citrate monohydrate and trisodium citrate dihydrate can be set to 0.01 to 100 of trisodium citrate dihydrate with respect to 1 of tripotassium citrate monohydrate. The mixing ratio may be set to about 1:1 as a molar ratio.

A mixing ratio of tripotassium citrate monohydrate ($C_6H_5K_3O_7 \cdot H_2O$), trisodium citrate dihydrate ($C_6H_5Na_3O_7 \cdot 2H_2O$), and anhydrous citric acid in the mixture of tripotassium citrate monohydrate ($C_6H_5K_3O_7 \cdot H_2O$), trisodium citrate dihydrate ($C_6H_5Na_3O_7 \cdot 2H_2O$), and anhydrous citric acid can be appropriately set by a person skilled in the art, and for example, the molar ratio of tripotassium citrate monohydrate, trisodium citrate dihydrate, and anhydrous citric acid can be set to 0.01 to 100 of trisodium citrate monohydrate and 0.01 to 100 of anhydrous citric acid with respect to 1 of tripotassium citrate monohydrate. The mixing ratio may be set to about 2:2:1 in a molar ratio.

In addition, other preferred examples of the active ingredient include sodium citrate or a hydrate thereof, and for example, the active ingredient may be trisodium citrate dihydrate ($C_6H_5Na_3O_7 \cdot 2H_2O$).

Furthermore, other preferred examples of the active ingredient include potassium citrate or a hydrate thereof, and for example, the active ingredient may be tripotassium citrate monohydrate ($C_6H_5K_3O_7 \cdot H_2O$).

In one embodiment, the active ingredient of the composition provided by the present invention may include a mixture of citric acid or a hydrate thereof, sodium citrate or a hydrate thereof and potassium citrate or a hydrate thereof, or may be composed only of a mixture of citric acid or a hydrate thereof, sodium citrate or a hydrate thereof and potassium citrate or a hydrate thereof.

In one embodiment, the active ingredient of the composition provided by the present invention may include a mixture of sodium citrate or a hydrate thereof and potassium citrate or a hydrate thereof, or may be composed only of a mixture of sodium citrate or a hydrate thereof and potassium citrate or a hydrate thereof.

In one embodiment, the active ingredient of the composition provided by the present invention is citric acid, a salt of citric acid, a hydrate thereof, or a mixture thereof, excluding ferrous citrate, ferric citrate, and hydrates thereof (for example, ferric citrate hydrate).

In the present specification, when the weight of citric acid or a hydrate thereof, a pharmaceutically acceptable salt of citric acid, or a hydrate thereof, or a mixture thereof (for example, tripotassium citrate monohydrate ($C_6H_5K_3O_7 \cdot H_2O$) and trisodium citrate dihydrate ($C_6H_5Na_3O_7 \cdot 2H_2O$)) is to be mentioned, the weight may be a dry weight.

As the composition provided by the present invention is administered or ingested, the muscle mass of the subject which has been administered with the composition or has ingested the composition increases. Examples of the muscle that increases include skeletal muscles. The muscle mass may be, for example, the weight of muscles per body weight.

In addition, as the composition provided by the present invention is administered or ingested, the renal function of the subject which has been administered with the composition or which has ingested the composition is ameliorated (for example, the creatinine clearance is ameliorated).

In the present specification, the increase in muscle mass, amelioration of renal function, suppression of a decrease in the muscle mass, and suppression of the deterioration of renal function can be checked by, for example, a comparison between the administration of the composition provided by the present invention and the administration of a placebo, and/or a comparison between before and after the administration of the composition provided by the present invention.

The subject of administration of the composition provided by the present invention may be a mammal (for example, a human) and is not particularly limited, and can be appropriately selected by a person skilled in the art.

The composition provided by the present invention may be administered to or taken in by a long-term bedridden person (for example, a long-term bedridden patient).

The disuse syndrome may be developed due to continuing a resting state for a long period of time for the treatment of a disease, an injury, or the like. The composition provided by the present invention can be used for the prevention or amelioration of disuse syndrome, particularly the prevention of deterioration of the physical ability seen in the disuse syndrome (for example, prevention of muscle weakness, prevention of muscle mass reduction, and prevention of the deterioration of renal function) or the amelioration of the physical ability of a human who has developed disuse syndrome (for example, amelioration of muscle strength, increase in muscle mass, and amelioration of renal function) or treatment. The composition provided by the present invention may be used in order to enhance the effect of rehabilitation for the purpose of ameliorating the physical ability.

In addition, the composition provided by the present invention may be administered to or taken in by an aged person (for example, 60 years or more of age, 65 years or more of age, 70 years or more of age, 75 years or more of age, 80 years or more of age, 90 years or more of age, 60 years or more and less than 75 years of age, 60 years or more and less than 90 years of age). The composition provided by the present invention can be used for the suppression of deterioration of the physical ability due to aging (for example, suppression of muscle weakness, suppression of muscle mass reduction, and prevention of the deterioration of renal function), and the amelioration of the physical ability that has deteriorated due to aging (for example, amelioration of muscle strength, increase in muscle mass, and amelioration of renal function).

In one embodiment, the composition provided by the present invention suppresses muscle weakness, suppresses muscle mass reduction, ameliorates muscle strength, or increases muscle mass, without requiring high-intensity exercise.

In one embodiment, the composition provided by the present invention suppresses the deterioration of renal function or ameliorates the renal function.

In one embodiment, the composition provided by the present invention suppresses muscle mass reduction and suppresses the deterioration of renal function or increases muscle mass, while ameliorating the renal function.

Citric acid, a salt thereof, a hydrate thereof, or a mixture thereof (for example, citric acid, sodium citrate dihydrate, and tripotassium citrate monohydrate), all of which are the active ingredients of the composition provided by the present invention, is a substance whose safety has been proven, and which may be administered or ingested without inducing harmful side effects on humans.

In the present specification, the "muscle mass" can be measured by, for example, a bioelectrical impedance analysis (BIA) method using a body composition analyzer (for example, InBody 770; manufactured by InBody Co., Ltd.).

In one embodiment, the increase in muscle mass can be represented by a comparison of administration of the composition provided by the present invention with respect to administration of a placebo or a control, and the following Calculation Formula (1) may be applied.

$$\text{Amount of increase in muscle mass } (\%) = [(\text{Muscle mass (g) after administration of composition provided by present invention - muscle mass after administration of placebo or control (g))} / \text{muscle mass after administration of placebo or control (g)}] \times 100 \qquad (1)$$

In one embodiment, the increase in muscle mass can be represented by a comparison of after the administration of the composition provided by the present invention with respect to before the administration of the composition provided by the present invention, and the following Calculation Formula (2) may be applied.

$$\text{Amount of increase in muscle mass } (\%) = [(\text{Muscle mass (g) after administration of composition provided by present invention - muscle mass (g) before administration of composition provided by present invention)} / \text{muscle mass (g) before administration of composition provided by present invention}] \times 100 \qquad (2)$$

In one embodiment, the composition provided by the present invention can be continuously administered for 6, 12, 24 weeks, or longer. For example, the amount of increase (%) in muscle mass after administration for 6 weeks of the composition provided by the present invention based on a comparison with the administration of a placebo or a control, may be 1% to 20%, 2% to 10%, 3% to 15%, 5% to 13%, 6% to 12%, 8% to 11%, or 9% to 10%, and the amount of increase (%) in muscle mass after administration for 6 weeks of the composition provided by the present invention based on a comparison between before and after the administration of the composition provided by the present invention, may be 1% to 20%, 2% to 10%, 3% to 15%, 5% to 13%, 6% to 12%, 8% to 11%, or 9% to 10%. For example, the amount of increase (%) in muscle mass after administration for 12 weeks of the composition provided by the present invention based on a comparison with the administration of a placebo or a control, may be 1% to 20%, 2% to 10%, 3% to 15%, 5% to 13%, 6% to 12%, 8% to 11%, or 9% to 10%, and the amount of increase (%) in muscle mass after administration for 12 weeks of the composition provided by the present invention based on a comparison between before and after the administration of the composition provided by the present invention, may be 1% to 20%, 2% to 10%, 3% to 15%, 5% to 13%, 6% to 12%, 8% to 11%, or 9% to 10%. For example, the amount of increase (%) in muscle mass after administration for 24 weeks of the composition provided by the present invention based on a comparison with the administration of a placebo or a control, may be 1 % to 20%, 2% to 10%, 3% to 15%, 5% to 13%, 6% to 12%, 8% to 11%, or 9% to 10%, and the amount of increase (%) in muscle mass after administration for 24 weeks of the composition provided by the present invention based on a comparison between before and after the administration of the composition provided by the present invention, may be 1% to 20%, 2% to 10%, 3% to 15%, 5% to 13%, 6% to 12%, 8% to 11%, or 9% to 10%.

The Calculation Formulas (1) and (2) for the amount of increase (%) in muscle mass may also be applied to the amount of decrease (%) in muscle mass. In this case, the amount of increase (%) in muscle mass is replaced with the amount of decrease (%) in muscle mass, and the amount of decrease (%) in muscle mass is expressed as a negative value by substituting each of the "muscle mass (g) after administration of composition provided by present invention", the "muscle mass (g) after administration of placebo or control", and the "muscle mass (g) before administration of composition provided by present invention".

In the present specification, the term "amelioration" is a concept encompassing bringing "pathological", "abnormal", or "uncomfortable" symptoms, conditions, or diseases closer to "healthy", "normal", or "comfortable" conditions, or action or means for that purpose; and converting them to "healthy", "normal", or "comfortable" conditions, or action or means for that purpose. Accordingly, in one embodiment, the term "amelioration" encompasses a concept in which a numerical value indicative of "pathological", "abnormal", or "uncomfortable" symptoms or conditions decreases or increases in accordance with the "amelioration" to become closer to a normal value or become a normal value. For example, amelioration of the renal function encompasses a concept in which the creatinine clearance decreases or increases to become closer to a normal value or become a normal value.

In the present specification, the term "treatment" is a concept encompassing eliminating, completely curing, healing, or palliating "pathological", "abnormal", or "uncomfortable" symptoms, conditions, or diseases, and action or means for that purpose; encompassing "suppressing" deterioration in the "pathological", "abnormal", or "uncomfortable" symptoms, conditions, or diseases, and action or means for that purpose; and encompassing "amelioration". In one embodiment, the term "treatment" encompasses eliminating, completely curing, healing, or palliating "pathological", "abnormal", or "uncomfortable" symptoms, conditions, or diseases, and action or means for that purpose. In another embodiment, the term "treatment" encompasses eliminating, completely curing, healing, or palliating "pathological", "abnormal", or "uncomfortable" symptoms, conditions, or diseases.

In the present specification, the term "suppression" is a concept encompassing stopping or slowing down of deterioration or progression of symptoms, conditions, or diseases, and action or means for that purpose; and encompassing ameliorating the symptoms, conditions, or diseases, or action or means for that purpose. The above-mentioned "deterioration or progression of symptoms, conditions, or diseases" includes deterioration or progression of "pathological", "abnormal", or "uncomfortable" symptoms, conditions, or diseases; and deterioration or progression from "healthy" or "normal" conditions to "pathological", "abnormal", or "uncomfortable" symptoms, conditions, or diseases. In one embodiment, the term "suppression" encompasses stopping or slowing down of deterioration or progression of symptoms, conditions, or diseases, or action or means for that purpose. In another embodiment, the term "suppression" encompasses stopping or slowing down of deterioration or progression of symptoms, conditions, or diseases.

In the present specification, the term "healthy" represents a state in which there is no acute or chronic disease or disorder, and the term "normal" represents a state which a healthy subject usually expresses.

In the present specification, the term "prevention" is a concept encompassing preventing onset of "pathological", "abnormal", or "uncomfortable" symptoms, conditions, or diseases before they occur, and action or means for that purpose.

In one embodiment, the composition provided by the present invention can be applied to the prevention of disuse syndrome; however, the composition may be ingested by or administered to a mammal (for example, a human) having a risk of continuing a resting state for a long period of time for treatment of a disease, an injury, or the like, in other words, a risk of long-term bedridden, from the initiation of the above-described resting state.

In the present specification, the term "long-term bedridden" means that "bed rest" lasts for a long period of time continuously or intermittently. In the present specification, in a case where the period of time for "bed rest" is longer than the period of time for "getting out of bed" in one day, the day is regarded as "bed rest". As one embodiment, "long-term bedridden" means that "bed rest" lasts for one week or longer continuously or intermittently; as another embodiment, "bed rest" lasts for 6 weeks or longer continuously or intermittently; as still another embodiment, "bed rest" lasts for 12 weeks or longer continuously or intermittently; and as still another embodiment, "bed rest" lasts for 24 weeks or longer continuously or intermittently. As one embodiment, the "long-term bedridden" means that "bed rest" lasts continuously for one week or longer; as another embodiment, "bed rest" lasts continuously for 6 weeks or longer; as still another embodiment, "bed rest" lasts continuously for 12 weeks or longer; and as still another embodiment, "bed rest" lasts continuously for 24 weeks or longer.

In the present specification, "enhancement of the effect of rehabilitation (also referred to as rehabilitation)" can be represented by a comparison of administration of the composition provided by the present invention with respect to administration of a placebo or a control, and for example, as described above, the amount of increase (%) in muscle mass can be used as an index.

In the present specification, the expression [A, B, and/or C] means that "at least one is selected from the group consisting of A, B, and C".

The composition provided by the present invention may be a pharmaceutical composition or a food composition.

A pharmaceutical composition provided by the present invention is orally or parenterally administered to a human or other mammals, and examples of parenteral administration include intravenous administration, subcutaneous administration, intramuscular administration, intra-articular administration, transmucosal administration, transdermal administration, transnasal administration, rectal administration, intrathecal administration, intraperitoneal administration, and topical administration.

The pharmaceutical composition provided by the present invention may be prepared by using citric acid, a salt of citric acid, a hydrate thereof, or a mixture thereof, as it is or by mixing with a pharmaceutically acceptable carrier, for example, an excipient (for example, lactose, D-mannitol, crystalline cellulose, or glucose), a binder (for example, hydroxypropyl cellulose (HPC), gelatin, or polyvinylpyrrolidone (PVP)), a lubricating agent (for example, magnesium stearate or talc), a disintegrant (for example, starch or carboxymethyl cellulose calcium (CMC-Ca)), a diluent (for example, water for injection or physiological saline), and other additives as necessary (for example, a pH adjuster, a surfactant, a dissolution aid, a preservative, an emulsifier, an isotonic agent, and a stabilizer), and the pharmaceutical composition may be a preparation such as a tablet, a capsule, a suspension, an injectable preparation, or a suppository. For example, in order to prepare as a tablet, citric acid, a pharmaceutically acceptable salt thereof, a hydrate thereof, or a mixture thereof may be formulated by mixing with an excipient (for example, lactose, D-mannitol, crystalline cellulose, or glucose), a disintegrant (for example, starch or carboxymethyl cellulose calcium (CMC-Ca)), a binder (for example, hydroxypropyl cellulose (HPC), gelatin, or polyvinylpyrrolidone (PVP)), a lubricating agent (for example, magnesium stearate or talc), and the like.

The dosage of the active ingredient, which is citric acid, a salt of citric acid, a hydrate thereof, or a mixture thereof, is appropriately determined according to the type of the active ingredient, the administration method, the age, body weight, gender, symptoms, sensitivity to drugs, and the like of the subject of administration; however, the dosage may be adjusted according to the circumstances of the amelioration of symptoms.

The present invention also provides a food composition including citric acid, a salt of citric acid, a hydrate thereof, or a mixture thereof.

The food composition provided by the present invention can exhibit an effect of enhancing the mass of muscles (for example, skeletal muscle) when ingested by a healthy person.

The food composition provided by the present invention may be ingested by an aged person (for example, 60 years or more of age, 65 years or more of age, 70 years or more of age, 75 years or more of age, 80 years or more of age, 90 years or more of age, 60 years or more and less than 75 years of age, or 60 years or more and less than 90 years of age). In addition, the food composition provided by the present invention may be ingested by a long-term bedridden person (for example, a long-term bedridden patient) or may be ingested to enhance the effect of rehabilitation intended for ameliorating the physical ability.

The content of citric acid, a salt of citric acid, a hydrate thereof, or a mixture thereof in the food composition provided by the present invention may be appropriately determined according to the type of the food. Examples of the food composition include foods for specified health use, dietary supplements, foods with function claims, foods for hospital patients, and supplements. The form of these food compositions is not particularly limited as long as it is a form that includes citric acid, a salt of citric acid, a hydrate thereof, or a mixture thereof in an effective amount for providing the above-mentioned effects and can be ingested orally, the form may be a form of an ordinary food or drink, or the food composition may be provided as a preparation suitable for oral administration, for example, a preparation such as a tablet, a capsule, or a suspension, among preparations that can be applied to the composition. Known formulation technologies can be applied to the configurations and production methods of these preparations.

For example, in the case of a food for specified health use, dietary supplements, a food with function claims, or a food for hospital patients, the food may include a 1/3 amount of 0.03 to 0.3 g of anhydrous citric acid and a 1/3 amount of a total of 1 to 3 g of tripotassium citrate monohydrate and trisodium citrate dihydrate per meal of the food. In a case where a food for specified health use, dietary supplements, a food with function claims, a food for hospital patients, or a supplement is provided as a tablet, for example, tablets of 300 mg to 600 mg per tablet may include 70% to 90% by weight of citric acid, a salt of citric acid, a hydrate thereof, or a mixture thereof.

In a case where the food composition provided by the present invention is not formulated and is provided in the form of an ordinary food or drink, the food composition can be appropriately produced by a person skilled in the art according to the type of the food, and for example, the food composition may be produced by blending citric acid, a salt of citric acid, a hydrate thereof, or a mixture thereof into a food material.

Examples of the form of the food and drink include liquid, milky, or pasty foods such as beverages, soy sauce, milk, yogurt, and miso; semi-solid foods such as jellies and gummies; solid foods such as candy, gums, tofu, and supplements; and powdered foods; and the like.

Examples of the beverages include fruit juice, fruit drinks, coffee drinks, oolong tea drinks, green tea drinks, black tea drinks, barley tea drinks, vegetable drinks, carbonated soft drinks, fruit extract drinks, vegetable extract juices, near water, sports drinks, diet drinks, and the like.

In beverages, additives such as antioxidants, fragrances, various esters, organic acids, organic acid salts, inorganic acids, inorganic acid salts, inorganic salts, color materials, emulsifiers, preservatives, seasonings, sweeteners, acidulants, fruit juice extracts, vegetable extracts, nectar extracts, pH adjusters, and quality stabilizers can be blended in singly or in combination.

In one embodiment, the composition provided by the present invention is a tablet, and one tablet may include 10 mg to 1 g, preferably 100 mg to 500 mg, and more preferably 400 mg to 500 mg, of tripotassium citrate monohydrate or trisodium citrate dihydrate as an alkalinizing agent.

In one embodiment, the composition provided by the present invention is a tablet, and one tablet may include tripotassium citrate monohydrate and trisodium citrate dihydrate in an amount of 10 mg to 300 mg for each and 20 mg to 600 mg in total, preferably 150 to 250 mg for each and 400 to 500 mg in total, and more preferably 190 to 240 mg for each and 400 to 450 mg in total.

In one embodiment, the composition provided by the present invention is a tablet, and one tablet includes 10 mg to 1 g, preferably 100 mg to 500 mg, and more preferably 400 mg to 500 mg, of tripotassium citrate monohydrate or trisodium citrate dihydrate as an alkalinizing agent and may include 10 mg to 500 mg, preferably 10 mg to 100 mg, and more preferably 50 mg to 100 mg, of anhydrous citric acid.

In one embodiment, the composition provided by the present invention is a tablet, and one tablet may include tripotassium citrate monohydrate and trisodium citrate dihydrate in an amount of 10 mg to 300 mg for each and 20 mg to 600 mg in total, preferably 150 to 250 mg for each and 400 to 500 mg in total, and more preferably 190 to 240 mg for each and 400 to 450 mg in total, and may include anhydrous citric acid in an amount of 10 mg to 500 mg, preferably 10 mg to 100 mg, and more preferably 50 mg to 100 mg.

In one embodiment, the composition provided by the present invention is a tablet, and the tablet includes, as active ingredients, 231.5 mg of tripotassium citrate monohydrate ($C_6H_5K_3O_7 \cdot H_2O$) and 195.0 mg of trisodium citrate dihydrate ($C_6H_5Na_3O_7 \cdot 2H_2O$) and may include anhydrous citric acid, crystalline cellulose, partially pregelatinized starch, hydroxypropyl cellulose, magnesium stearate, Hypromellose, Macrogol 6000, titanium oxide, and carnauba wax as additives. In this embodiment, the tablet provided by the present invention may include 72.5 mg of anhydrous citric acid as an additive.

The dosage or intake amount of the active ingredient included in the composition provided by the present invention can be appropriately set by a person skilled in the art.

In one embodiment, in a case where the active ingredient is a mixture of tripotassium citrate monohydrate and trisodium citrate dihydrate, tripotassium citrate monohydrate and trisodium citrate dihydrate may be administered or ingested at a dosage or intake amount of 0.1 to 5 g/day for each and 0.2 to 10 g/day in total, 0.1 to 3 g/day for each and 0.2 to 6 g/day in total, 0.5 to 3 g/day for each and 1 to 6 g/day in total, preferably 0.5 to 1.5 g/day for each and 1 to 3 g/day in total, 1 to 1.5 g/day for each 2 to 3 g/day in total, or 0.5 to 1 g/day for each and 1 to 2 g/day in total, and may be administered or ingested in one to five separate parts a day, and preferably three separate parts a day.

In one embodiment, in a case where the active ingredient is tripotassium citrate monohydrate or trisodium citrate dihydrate, the active ingredient may be administered or ingested at a dosage or intake amount of 1 to 10 g/day, 1 to 6 g/day, 2 to 5.5 g/day, 1 to 3 g/day, 2 to 3 g/day, or 1 to 1.5 g/day, and may be administered or ingested in one to five separate parts a day, and preferably three separate parts a day.

In one embodiment, in a case where the active ingredient is a mixture of anhydrous citric acid, tripotassium citrate monohydrate, and trisodium citrate dihydrate, anhydrous citric acid may be administered or ingested at a dosage or intake amount of 0.1 to 2 g/day, preferably 0.1 to 1 g/day, and more preferably 0.3 to 0.6 g/day; and tripotassium citrate monohydrate and trisodium citrate dihydrate may be administered or ingested at a dosage or intake amount of 0.1 to 5 g/day for each and 0.2 to 10 g/day in total, 0.1 to 3 g/day for each and 0.2 to 6 g/day in total, 0.5 to 3 g/day for each and 1 to 6 g/day in total, preferably 0.5 to 1.5 g/day for each and 1 to 3 g/day in total, 1 to 1.5 g/day for each and 2 to 3 g/day in total, or 0.5 to 1 g/day for each and 1 to 2 g/day in total, and may be administered or ingested in one to five separate parts a day, and preferably three separate parts a day.

In one embodiment, in a case where the active ingredient is anhydrous citric acid, the active ingredient may be administered or ingested at a dose or intake amount of 0.1 to 2 g/day, preferably 0.1 to 1 g/day, and more preferably 0.3 to 0.6 g/day, and may be administered or ingested one to five separate parts a day, and preferably three separate parts a day.

The duration for administration or ingestion of the composition provided by the present invention may be appropriately set, and for example, the duration may be 5 days, 1 week, 2 weeks, 1 month, 5 days or more and 10 days or less, 1 week or more and 2 weeks or less, 1 week or more and 1 month or less, 5 days or more, 1 week or more, 2 weeks or more, or 1 month or more.

Examples of embodiments provided by the present invention include the following.

(1-1)
Use of citric acid, a salt of citric acid, a hydrate thereof, or a mixture thereof, for producing a composition for increasing muscle mass;
(1-2)
Use of citric acid, a salt of citric acid, a hydrate thereof, or a mixture thereof, for producing a composition for preventing disuse syndrome;
(1-3)
Use of citric acid, a salt of citric acid, a hydrate thereof, or a mixture thereof, for producing a composition for enhancing an effect of rehabilitation of a long-term bedridden person;
(1-4)
Use of citric acid, a salt of citric acid, a hydrate thereof, or a mixture thereof, for producing a composition for suppressing a decrease in muscle mass of an aged person;

(1-5)

Use of citric acid, a salt of citric acid, a hydrate thereof, or a mixture thereof, for producing a composition for ameliorating renal function of a long-term bedridden person or an aged person;

(2-1)

A composition including citric acid, a salt of citric acid, a hydrate thereof, or a mixture thereof, for use in increasing muscle mass;

(2-2)

A composition including citric acid, a salt of citric acid, a hydrate thereof, or a mixture thereof, for use in preventing disuse syndrome;

(2-3)

A composition including citric acid, a salt of citric acid, a hydrate thereof, or a mixture thereof, for use in enhancing an effect of rehabilitation of a long-term bedridden person;

(2-4)

A composition including citric acid, a salt of citric acid, a hydrate thereof, or a mixture thereof, for use in suppressing a decrease in muscle mass in an aged person;

(2-5)

A composition including citric acid, a salt of citric acid, a hydrate thereof, or a mixture thereof, for use in ameliorating renal function of a long-term bedridden person or an aged person;

(3-1)

A method for increasing muscle mass, the method including administering a composition including an effective amount of citric acid, a salt of citric acid, a hydrate thereof, or a mixture thereof, to a subject in need of an increase in muscle mass;

(3-2)

A method for preventing disuse syndrome, the method including administering a composition including an effective amount of citric acid, a salt of citric acid, a hydrate thereof, or a mixture thereof, to a subject in need of prevention of disuse syndrome;

(3-3)

A method for enhancing an effect of rehabilitation of a long-term bedridden person, the method including administering a composition including an effective amount of citric acid, a salt of citric acid, a hydrate thereof, or a mixture thereof, to a subject in need of enhancement of an effect of rehabilitation;

(3-4)

A method for suppressing a decrease in muscle mass in an aged person, the method including administering a composition including an effective amount of citric acid, a salt of citric acid, a hydrate thereof, or a mixture thereof, to a subject in need of suppression of a decrease in muscle mass;

(3-5)

A method for ameliorating renal function of a long-term bedridden person or an aged person, the method including administering a composition including an effective amount of citric acid, a salt of citric acid, a hydrate thereof, or a mixture thereof, to a subject in need of amelioration of renal function;

(4-1)

The use, composition, or method according to any one of (1-1) to (1-5), (2-1) to (2-5), and (3-1) to (3-5), in which the citric acid, the salt of citric acid, the hydrate thereof, or the mixture thereof, is an alkali metal salt of citric acid;

(4-2)

The use, composition, or method according to any one of (1-1) to (1-5), (2-1) to (2-5), and (3-1) to (3-5), in which the citric acid, the salt of citric acid, the hydrate thereof, or the mixture thereof, is sodium citrate or a hydrate thereof, potassium citrate or a hydrate thereof, or a mixture thereof;

(4-3)

The use, composition, or method according to any one of (1-1) to (1-5), (2-1) to (2-5), and (3-1) to (3-5), in which the citric acid, the salt of citric acid, the hydrate thereof, or the mixture thereof, is sodium citrate or a hydrate thereof;

(4-4)

The use, composition, or method according to any one of (1-1) to (1-5), (2-1) to (2-5), (3-1) to (3-5), and (4-1) to (4-5), in which the citric acid, the salt of citric acid, the hydrate thereof, or the mixture thereof, is a mixture of trisodium citrate dihydrate and tripotassium citrate monohydrate;

(4-5)

The use, composition, or method according to any one of (1-1) to (1-5), (2-1) to (2-5), (3-1) to (3-5), and (4-1) to (4-5), in which the citric acid, the salt of citric acid, the hydrate thereof, or the mixture thereof, is a mixture of trisodium citrate dihydrate and tripotassium citrate monohydrate, and an oral dosage or intake amount is 0.5 g to 1.5 g/day for each and 1 to 3 g/day in total;

(4-6)

The use, composition, or method according to any one of (4-1) to (4-5), further including citric acid;

(4-7)

The use, composition, or method according to any one of (1-1) to (1-5), (2-1) to (2-5), (3-1) to (3-5), and (4-1) to (4-6), in which the citric acid, the salt of citric acid, the hydrate thereof, or the mixture thereof, is a mixture of citric acid, trisodium citrate dihydrate, and tripotassium citrate monohydrate, and a weight ratio of the mixture is 2:2:1;

(4-8)

The use, composition, or method according to any one of (1-1) to (1-5), (2-1) to (2-5), (3-1) to (3-5), and (4-1) to (4-7), in which the composition is a food;

(4-9)

The use, composition, or method according to any one of (1-1) to (1-5), (2-1) to (2-5), (3-1) to (3-5), and (4-1) to (4-8), in which the composition is a food composition whose effect of increasing muscle mass is indicated on packaging, a container, or an instruction leaflet; and

(4-10)

The use, composition, or method according to any one of (1-1) to (1-5), (2-1) to (2-5), (3-1) to (3-5), and (4-1) to (4-9), in which the citric acid, the salt of citric acid, the hydrate thereof, or the mixture thereof, is a mixture of trisodium citrate dihydrate and tripotassium citrate monohydrate, an oral dosage or intake amount is 0.5 g to 1.5 g/day for each and 1 to 3 g/day in total, and an increase in muscle mass and/or amelioration of renal function is recognized as a result of administration or ingestion.

Hereinafter, the present invention will be further described by way of Examples; however, the present invention is not intended to be limited to these.

[Examples]

The following experiment was performed for the purpose of checking a muscle mass increasing effect of a mixture of sodium citrate, potassium citrate, and citric acid.

Male Crl:CD(SD) rats (8 weeks of age at the time of initiation of administration) were divided into two groups, and an aqueous solution of a test drug (group D) or purified water (group C) was orally administered forcibly and repeatedly twice a day for 7 days.

The dosage volume of the control group (group C) was set to 10.0 mL/kg, and for the test drug administration group (group D), the dosage was set to 2000 mg/kg, the concentration was set to 100 mg/mL, and the dosage volume was set to 10.0 mL/kg. An autopsy was performed on the day after the 7th day of administration.

The above-described aqueous solution of the test drug was produced as follows.

463 mg of tripotassium citrate monohydrate ($C_6H_5K_3O_7 \cdot H_2O$), 390 mg of trisodium citrate dihydrate ($C_6H_5Na_3O_7 \cdot 2H_2O$), and 145 mg of anhydrous citric acid were dissolved in purified water, and adjusted to 100 mg/mL.

As a result, in group D, the weight of the vastus lateralis muscle per 100 g of the body weight was significantly higher as compared with group C (FIG. 1). For statistical analysis, an unpaired t test was used.

In addition, a metabolome analysis was performed on the muscles collected at the time of autopsy on the day after the 7th day of administration.

After receiving a rat muscle tissue sample, 750 $\mu$L of a 50% aqueous solution of acetonitrile (v/v) (internal standard substance concentration: 20 $\mu$M) was added to the sample, the resulting mixture was crushed under cooling using a crusher (3,500 rpm, 60 seconds $\times$ 5 times), and the same amount of a 50% aqueous solution of acetonitrile (v/v) was further added to the sample. After the tissue disruption, centrifugal separation (2,300$\times$g, 4°C, for 5 minutes) was performed. After the centrifugal separation, 400 $\mu$L of an upper layer was transferred into an ultrafiltration tube (ULTRAFREE MC PLHCC, HMT, centrifugal filter unit 5 kDa). This was centrifuged (9,100$\times$g, 4°C, for 120 minutes), and an ultrafiltration treatment was performed. The filtrate was dried and solidified, dissolved again in 50 $\mu$L of Milli-Q water, and submitted to measurement. Measurement was carried out in a cation mode and an anion mode of CE-TOFMS and CE-QqQMS. As measurement targets, 116 (cation 52, anion 64) kinds of metabolites occupying important roles in the glycolysis system, the pentose phosphate pathway, the citric acid cycle, the urea cycle, the polyamine creatine metabolic pathway, the purine metabolic pathway, the glutathione metabolic pathway, the nicotinamide metabolic pathway, the choline metabolic pathway, and various amino acid metabolic pathways, were selected and analyzed. Absolute quantitative values of these metabolites were calculated, and each substance was drawn on the metabolic pathway of the substance. Among the results, items (NADPH/NADP$^+$, Fischer's Ratio) for which $p < 0.05$ in the unpaired t test are shown in Table 1. All of these are items related to energy production or protein synthesis/degradation. Therefore, it is conceivable that due to the energy production increased and the promotion of protein synthesis or/and suppression of protein degradation occurred as a result of ingestion of citrate, the relative muscle mass was higher in the group D as compared with the group C.

When the weight of the rat vastus lateralis muscle was measured, the muscle mass per body weight was significantly increased in the group D as compared with the group C (FIG. 1). Since no significant difference in body weight was recognized between the group C and the group D, these results rule out that the muscle mass increased due to the increase in the body weight. Furthermore, the creatinine clearance (Ccr) per rat body weight was measured, and in the group D, the Ccr increased after administration of the aqueous solution of the test drug as compared with the group C (FIG. 2). The Ccr was calculated from the serum creatinine values before administration and on the day of autopsy, the 21-hour urinary creatinine, and the body weight. Specifically, after unit conversion, the Ccr was calculated by dividing the amount of urinary creatinine per minute by the serum creatinine and the body weight. A paired t test was used for changes between before administration and on the autopsy day, and an unpaired t test was used for tests between groups. These results support the results of the increase in muscle mass brought by administration of the aqueous solution of the test drug (FIG. 1). The Ccr itself is an index of renal function. However, it is known that deterioration of renal function leads to a decrease in muscle mass, and an increase in muscle mass leads to a kidney protection effect. It is conceivable that there is a possibility that the renal function was also improved through this relationship between muscles and the kidney. In addition, since the serum creatinine is considered to be affected by muscle mass, it is predicted that the serum creatinine in the group C will be higher than that in the group D. However, in the present test, although the relative muscle mass in the group C was significantly higher than that in the group D, no significant difference was observed in the serum creatinine. From this as well, it is considered that the renal function in the group C was improved as compared with that in the group D. As described above, it is understood that the ingestion of citric acid salts in the present test ameliorated the functions of muscles and kidneys through the muscle-kidney linkage.

[Table 1]

Table 1. Results of metabolome analysis in muscle tissue ($p < 0.05$)

| Metabolite | Control | | Citrate | | p-value |
|---|---|---|---|---|---|
| | mean $\pm$ SD | n | mean $\pm$ SD | n | |
| NADPH/NADP$^+$ | 1.599 $\pm$ 0.431 | 6 | 1.089 $\pm$ 0.194 | 7 | 0.0163* |
| Fischer's Ratio | 2.045 $\pm$ 0.192 | 6 | 1.768 $\pm$ 0.193 | 7 | 0.0253* |

unit: nmol/g of wet tissue, *$p < 0.05$ (unpaired t test),
Fischer's ratio: Ratio of branched amino acids / aromatic amino acids

[Industrial Applicability]

The composition and the like provided by the present invention may increase muscle mass in a mammal, ameliorate renal function, and reduce the burden on the nursing care field for aged people and long-term bedridden people.

**Claims**

1. A composition for increasing muscle mass in a mammal, the composition comprising citric acid, a salt of citric acid, a hydrate thereof, or a mixture thereof.

2. The composition according to Claim 1, wherein the composition is a food composition.

3. The composition according to Claim 1 or 2, wherein the citric acid, the salt of citric acid, the hydrate thereof, or the mixture thereof is an alkali metal salt of citric acid.

4. The composition according to Claim 1 or 2, wherein the citric acid, the salt of citric acid, the hydrate thereof, or the mixture thereof is a mixture of sodium citrate or a hydrate thereof and potassium citrate or a hydrate thereof.

5. The composition according to Claim 3, further comprising anhydrous citric acid.

6. The composition according to Claim 2, wherein the composition is a food composition in which an effect of increasing muscle mass is indicated on packaging, a container, or an instruction leaflet.

7. The composition according to Claim 1 or 2, wherein the composition is used for preventing disuse syndrome.

8. The composition according to Claim 1 or 2, wherein the composition is used for enhancing an effect of rehabilitation of a long-term bedridden person.

9. The composition according to Claim 1 or 2, wherein the composition is used for suppressing a decrease in muscle mass of an aged person.

10. The composition according to Claim 1 or 2, wherein the composition is a tablet.

11. The composition according to Claim 1 or 2, wherein the composition ameliorates renal function of a mammal.

FIG. 1

**muscle relative weight**

relative weight [g/100 g B.W.]

*

Control    Citrate

Mean±SD

*p=0.0306 (unpaired t test)

$$\text{relative weight [g/100 g B.W.]} = \frac{\text{muscle weight [g]}}{\text{body weight [g]}} \times 100$$

FIG. 2

**Creatinine clearance**

Mean±SD
**p=0.0011 (paired t test)
Not significant between groups (unpaired t test)

$$\text{Ccr [mL/min/kg B.W.]} = \frac{\text{Ucr [mg/min]}}{\text{Scr [mg/mL]} \times \text{BODY WEIGHT[kg]}}$$

Ucr: URINARY CREATININE, Scr: SERUM CREATININE

## INTERNATIONAL SEARCH REPORT

| International application No. |
|---|
| **PCT/JP2022/019747** |

**A. CLASSIFICATION OF SUBJECT MATTER**

*A61K 31/194*(2006.01)i; *A23L 33/10*(2016.01)i; *A61K 9/20*(2006.01)i; *A61P 13/12*(2006.01)i; *A61P 17/00*(2006.01)i; *A61P 19/02*(2006.01)i; *A61P 21/00*(2006.01)i

FI: A61K31/194; A23L33/10; A61K9/20; A61P13/12; A61P17/00; A61P19/02; A61P21/00

According to International Patent Classification (IPC) or to both national classification and IPC

**B. FIELDS SEARCHED**

Minimum documentation searched (classification system followed by classification symbols)

A61K31/194; A23L33/10; A61K9/20; A61P13/12; A61P17/00; A61P19/02; A61P21/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2018-153166 A (POKKA SAPPRO FOOD & BEVERAGE LTD) 04 October 2018 (2018-10-04) claims, paragraphs [0015], [0018], [0020], [0022]-[0025], examples, table 1 | 1-11 |
| X | JP 2019-189571 A (UNIV TOKUSHIMA) 31 October 2019 (2019-10-31) claims, paragraphs [0028], [0029], example 1, reference examples | 1-11 |
| Y | JP 2020-528400 A (PROFESSIONAL DIETETICS INTERNATIONAL S.R.L) 24 September 2020 (2020-09-24) claims, paragraphs [0008], [0061], examples | 1-11 |
| Y | COEN, Paul M. et al. Mitochondria as a Target for Mitigating Sarcopenia. Frontiers in Physiology. 2019, vol. 9, article 1883, pp. 1-15, DOI: 10.3389/fphys.2018.01883 abstract, fig. 1, summary | 1-11 |
| Y | WO 2020/080499 A1 (TOHOKU UNIVERSITY) 23 April 2020 (2020-04-23) claims | 11 |

☑ Further documents are listed in the continuation of Box C. ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **11 July 2022** | **26 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| | International application No. |
| --- | --- |
| | **PCT/JP2022/019747** |

**C.    DOCUMENTS CONSIDERED TO BE RELEVANT**

| Category* | Citation of document, with indication, where appropriate, of the relevant passages | Relevant to claim No. |
| --- | --- | --- |
| Y | WO 2018/193752 A1 (TOHOKU UNIVERSITY) 25 October 2018 (2018-10-25)<br>claims | 11 |
| A | JP 2021-63059 A (UHA MIKAKUTO CO LTD) 22 April 2021 (2021-04-22)<br>claims, etc. | 1-11 |
| A | JP 2019-19105 A (OTSUKA PHARMA FACTORY INC) 07 February 2019 (2019-02-07)<br>claims, etc. | 1-11 |

**INTERNATIONAL SEARCH REPORT**
**Information on patent family members**

International application No.

**PCT/JP2022/019747**

| Patent document cited in search report | | | Publication date (day/month/year) | Patent family member(s) | | | Publication date (day/month/year) |
|---|---|---|---|---|---|---|---|
| JP | 2018-153166 | A | 04 October 2018 | (Family: none) | | | |
| JP | 2019-189571 | A | 31 October 2019 | (Family: none) | | | |
| JP | 2020-528400 | A | 24 September 2020 | US claims, paragraphs [0008], [0060], examples | 2020/0230093 | A1 | |
| | | | | EP | 3915553 | A1 | |
| | | | | CN | 110996935 | A | |
| | | | | KR | 10-2020-0033872 | A | |
| WO | 2020/080499 | A1 | 23 April 2020 | US claims | 2021/0386696 | A1 | |
| | | | | EP | 3868371 | A1 | |
| | | | | CN | 112888434 | A | |
| | | | | KR | 10-2021-0078499 | A | |
| WO | 2018/193752 | A1 | 25 October 2018 | US claims | 2021/0121426 | A1 | |
| | | | | EP | 3616721 | A1 | |
| | | | | CN | 110799214 | A | |
| | | | | KR | 10-2019-0137147 | A | |
| JP | 2021-63059 | A | 22 April 2021 | (Family: none) | | | |
| JP | 2019-19105 | A | 07 February 2019 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021080076 A
- JP 2016136952 A
- JP 2018153166 A